# EUROPEAN PATENT APPLICATION

(11) **EP 3 708 222 A1**
(43) Date of publication of application: **16.09.2020**
(21) Application number: 18875045.9
(22) Date of filing: 07.11.2018
(51) Int. Cl.: A61N 1/44, A61C 1/00, A61C 17/00, H05H 1/24

(54) **PLASMA-TYPE TREATMENT DEVICE**

(30) Priority: 08.11.2017 JP 2017215732
(71) Applicant: Sekisui Chemical Co., Ltd., Osaka-shi, Osaka 530-8565 (JP)
(72) Inventor: NAGAHARA Yu, Kyoto-shi Kyoto 601-8105 (JP); UEHARA Tsuyoshi, Kyoto-shi Kyoto 601-8105 (JP); OSHITA Takaya, Kyoto-shi Kyoto 601-8105 (JP); TAKIKAWA Yoshishige, Kyoto-shi Kyoto 601-8105 (JP)
(74) Representative: Ter Meer Steinmeister & Partner
(86) International application number: PCT/JP2018/041340
(87) International publication number: WO 2019/093375

(57) **Abstract**

The plasma application therapeutic apparatus (100) of the present invention includes: a plasma generating unit, a nozzle for discharging at least one of plasma generated by the plasma generating unit and a reactive gas generated by the plasma, a supply source (70) for supplying a plasma generating gas to the plasma generating unit, an operation unit (9) which is configured to be activated by a user to allow the supply source(70) to supply a predetermined amount of the plasma generating gas to the plasma generating unit, and a reporting unit (80) which is configured to report a remaining gas information in terms of remaining number of times allowed for the supply source (70) to supply the plasma generating gas to the plasma generating unit, based on the plasma generating gas remaining in the supply source.

## Description

### TECHNICAL FIELD

The present invention relates to a plasma application therapeutic apparatus. Priority is claimed on Japanese Patent Application No. 2017-215732, filed November 8, 2017, the contents of which are incorporated herein by reference.

### BACKGROUND ART

Conventionally, a plasma application therapeutic apparatus for medical use such as dental treatment has been known. The plasma application therapeutic apparatus cures the affected area by applying plasma or reactive gas to the affected area such as wounds. The reactive gas is generated by plasma in a plasma application therapeutic apparatus. For example, Patent Document 1 discloses a plasma jet application apparatus for implementing dental treatment. The plasma jet application apparatus is equipped with an application instrument having a plasma jet application means. The plasma jet application apparatus generates plasma and applies the generated plasma together with reactive species to a target object. The reactive species are generated by reaction of the plasma with the gas present within or around the plasma.

Patent Document 2 discloses a plasma application therapeutic apparatus that generates reactive gas (reactive species) inside an application instrument, and discharges the reactive gas from the nozzle of the application instrument to apply the reactive gas to an affected area of a patient. The reactive gas is, for example, active oxygen or active nitrogen.

### Description of Prior Art

### Patent Document

Patent Document 1: Japanese Patent Granted Publication No. 5441066
Patent Document 2: Japanese Unexamined Patent Application Publication No. 2017-50267

### DISCLOSURE OF INVENTION

### Problems to be Solved by the Invention

As regards the conventional plasma application therapeutic apparatus, there is a room for improvement in usability for doctors, etc. In particular, the supply source of the plasma generating gas is usually an exchangeable cylinder or the like, and the plasma generating gas results in being wasted if the supply source is replaced while the plasma generating gas is still remaining in the supply source. In addition, the plasma application therapeutic apparatus itself seemingly operates normally even if the plasma generating gas in the supply source has been completely consumed, so that the plasma application therapeutic apparatus may be kept on being used unintentionally in spite of lack of the plasma generating gas in the supply source. This not only results in failure to obtain desired therapeutic effect, but also may pose a risk that a large amount of energy is caused to be wasted due to a large amount of electric power required to operate the plasma application therapeutic apparatus. This issue of energy wastage can also be a particularly acute problem when using portable power supplies.

Thus, there has been a risk that too early replacement of the supply source due to inability to accurately grasp the remaining amount of plasma generating gas may result in waste of the gas, while too late replacement could lead to undesirable consequences of inadequate therapeutic effect and waste of valuable electricity.

Further, in the field of plasma application therapy, there is a technique for deliberately controlling the reactive species produced by adding a small amount of a second gas to the main plasma generating gas. When this technique is used, it is necessary to install two types of cylinders on a plasma application therapeutic apparatus. In the case where different amounts of gases are used or the amount of a second gas is varied depending on the type of therapy, it is very difficult to accurately grasp the remaining amounts of the gases with conventional regulators that regulate pressure based on stored data.

The present invention has been made in view of the circumstances as described above, and the object of the invention is to improve the usability of a plasma application therapeutic apparatus by preventing the waste of plasma generating gas and electric power, and by ensuring the therapeutic effect.

### Means to Solve the Problems

Embodiments proposed by the present invention in order to solve the above-mentioned problem are as enumerated below.

The plasma application therapeutic apparatus of the present invention includes: a plasma generating unit, a nozzle for discharging at least one of plasma generated by the plasma generating unit and a reactive gas generated by the plasma, a supply source for supplying a plasma generating gas to the plasma generating unit, an operation unit which is configured to be activated by a user to allow the supply source to supply a predetermined amount of the plasma generating gas to the plasma generating unit, and a reporting unit which is configured to report a remaining gas information in terms of remaining number of times allowed for the supply source to supply the plasma generating gas to the plasma generating unit, based on the plasma generating gas remaining in the supply source.

In this instance, the reporting unit reports the remaining number of times for supplying the plasma generating gas. Therefore, for example, the user can easily tell the timing of replacement of the supply source, and the usability of the plasma application therapeutic apparatus can be improved.

The plasma application therapeutic apparatus may further includes a calculation unit configured to calculate the remaining number of times, based on a remaining amount of the plasma generating gas in the supply source and a supply amount of the plasma generating gas per operation of the operation unit.

In this instance, the calculation calculates the remaining number of times for supplying the plasma generating gas, based on a remaining amount of the plasma generating gas in the supply source and a supply amount of the plasma generating gas per operation of the operation unit. This can improve the accuracy of the remaining number of times to be reported.

The plasma application therapeutic apparatus of the present invention includes: a plasma generating unit, a nozzle for discharging at least one of plasma generated by the plasma generating unit and a reactive gas generated by the plasma, a supply source for supplying a plasma generating gas to the plasma generating unit, and a reporting unit which is configured to report a remaining gas information in terms of remaining time allowed for the supply source to supply the plasma generating gas to the plasma generating unit, based on the plasma generating gas remaining in the supply source.

In this instance, the reporting unit reports the remaining time for supplying the plasma generating gas. Therefore, for example, the user can easily tell the timing of replacement of the supply source for the plasma generating gas, and the usability of the plasma application therapeutic apparatus can be improved.

The plasma application therapeutic apparatus may further includes a calculation unit configured to calculate the remaining time, based on a remaining amount of the plasma generating gas in the supply source and a supply amount of the plasma generating gas per unit time.

In this instance, the calculation unit calculates the remaining time for supplying the plasma generating gas, based on a remaining amount of the plasma generating gas in the supply source and a supply amount of the plasma generating gas per unit time. This can improve the accuracy of the remaining time to be reported.

The reporting unit may display the remaining gas information.

In this instance, the reporting unit displays the remaining gas information. Therefore, for example, the user can see the information on the remaining plasma generating gas, unlike the case in which the reporting unit announces the remaining gas information by voice.

The supply source may include two or more cylinders which are configured to respectively supply different plasma generating gases to the plasma generating unit.

In this instance, the supply source includes two or more cylinders, which respectively supply different plasma generating gases to the plasma generating unit. Therefore, it is possible to improve the accuracy of the remaining gas information of each cylinder by having the reporting unit report the remaining gas information on the remaining number of times or retaining time for allowing each cylinder to supply the plasma generating gas to the plasma generating unit.

### Effect of the Invention

The present invention allows for improvement in the usability of a plasma application therapeutic apparatus by preventing the waste of plasma generating gas and electric power, and by ensuring the therapeutic effect.

### BRIEF DESCRIPTION OF THE DRAWINGS

FIG. 1 is a schematic view showing a plasma application therapeutic apparatus according to one embodiment of the present invention.
FIG. 2 is a partial cross-sectional view showing an application instrument included in the plasma application therapeutic apparatus according to one embodiment of the present invention.
FIG. 3 is a cross-sectional view showing the application instrument of FIG. 2 as viewed from the arrow direction of the x-x line of FIG. 2.
FIG. 4 is a cross-sectional view showing the application instrument of FIG. 2 as viewed from the arrow direction of the y-y line of FIG. 2.
FIG. 5 is block diagram showing a schematic configuration of a plasma application therapeutic apparatus according to one embodiment of the present invention.
FIG. 6 is a schematic view showing an example of modification of the plasma application therapeutic apparatus of the present invention.

### DESCRIPTION OF THE EMBODIMENTS

The plasma application therapeutic apparatus of the present invention is a plasma jet application apparatus or a reactive gas application apparatus.

The plasma jet application apparatus generates plasma. The plasma jet application apparatus generates plasma and applies the generated plasma together with reactive species to a target object. The reactive species are generated by reaction of the plasma with the gas present within or around the plasma. Examples of the reactive species include reactive oxygen species and reactive nitrogen species. Examples of the reactive oxygen species include hydroxyl radicals, singlet oxygen, ozone, hydrogen peroxide, and superoxide anion radicals. Examples of the reactive nitrogen species include nitric oxide, nitrogen dioxide, peroxynitrite, peroxynitrite, and dinitrogen trioxide.

The reactive gas application apparatus generates plasma. The reactive gas application apparatus applies the reactive gas containing the reactive species to a target object. The reactive species are generated by reaction of the plasma with the gas present within or around the plasma.

One embodiment of the plasma application therapeutic apparatus of the present invention is described below.
The plasma application therapeutic apparatus of the present embodiment is a reactive gas application apparatus.

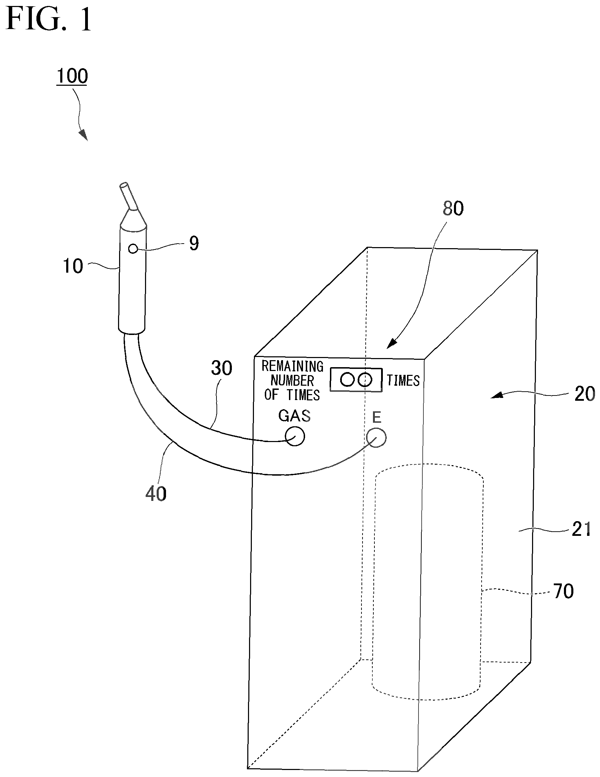

As shown in FIGs. 1 to 5, the reactive gas application apparatus 100 of the present embodiment has an application instrument 10, a detection unit 15, a supply unit 20, a gas conduit 30, an electric wiring 40, a supply source 70, a reporting unit 80, and a controller unit 90 (calculation unit).

The application instrument 10 discharges the reactive gas generated in the application instrument 10. The supply unit 20 supplies electric power and plasma generating gas to the application instrument 10. The supply unit 20 houses the supply source 70. The supply source 70 contains the plasma generating gas. The supply unit 20 is connected to a power supply (not shown), such as a 100 V household power supply. In another one of the preferred embodiments of the present invention, the power supply is a portable power supply. The gas conduit 30 connects the application instrument 10 with the supply unit 20. The electrical wiring 40 connects the application instrument 10 with the supply unit 20. In the present embodiment, the gas conduit 30 and the electric wiring 40 are provided independently from each other, but the gas conduit 30 and the electric wiring 40 may be integrated.

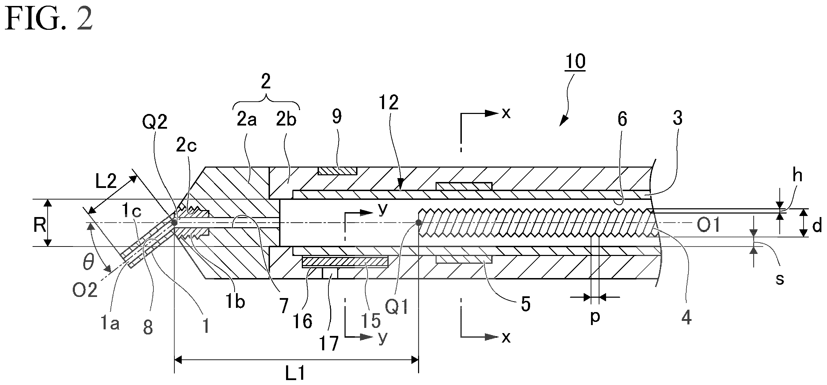

FIG. 2 is a cross-sectional view (longitudinal section) showing a plane along the axis of the application instrument 10.

As shown in FIG. 2, the application instrument 10 includes an elongated cowling 2, a nozzle 1 protruding from the tip of the cowling 2, and a plasma generating unit 12 provided in the cowling 2.

The cowling 2 includes a cylindrical body 2b and a head 2a covering the tip of the body 2b. The body 2b is not limited to that of a cylindrical shape, and may be of a polygonal tube shape such as a square tube shape, a hexagonal tube shape, an octagonal tube shape or the like.

The head 2a gradually narrows toward the tip thereof. That is, the head 2a in the present embodiment has a conical shape. The head 2a is not limited to that of a conical shape, and may be of a polygonal cone shape such as a quadrangular pyramid shape, a hexagonal pyramid shape, an octagonal pyramid shape or the like. The head 2a has a fitting hole 2c at its tip. The fitting hole 2c is a hole for receiving the nozzle 1. The nozzle 1 is detachably attached to the head 2a. A first reactive gas flow path 7 extending in the tube axis O1 direction is provided inside the head 2a. The tube axis O1 is a tube axis of the body 2b.

The body 2b has an operation switch 9 (operation unit) on its outer peripheral surface.

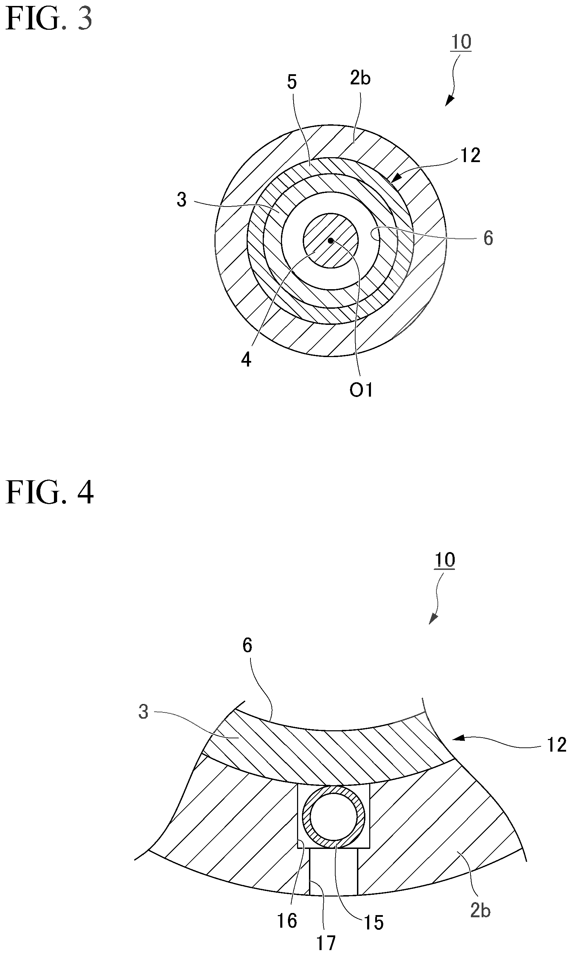

As shown in FIGs. 2 and 3, the plasma generating unit 12 has a tubular dielectric 3 (dielectric), an inner electrode 4, and an outer electrode 5.

The tubular dielectric 3 is a cylindrical member extending in the tube axis O1 direction. The tubular dielectric 3 has in its inside a gas flow path 6 extending in the tube axis O1 direction. The gas flow path 6 communicates with a first reactive gas flow path 7. The tube axis O1 coincides with the tube axis of the tubular dielectric 3.

In the tubular dielectric 3, an inner electrode 4 is provided. The inner electrode 4 is a substantially columnar member extending in the tube axis O1 direction. The inner electrode 4 is spaced apart from the inner surface of the tubular dielectric 3.

On the outer peripheral surface of the tubular dielectric 3, an outer electrode 5 extending along the inner electrode 4 is provided. The outer electrode 5 is an annular electrode that surrounds the outer peripheral surface of the tubular dielectric 3.

As shown in FIG. 3, the tubular dielectric 3, the inner electrode 4, and the outer electrode 5 are positioned concentrically around the tube axis O1.

In the present embodiment, the outer peripheral surface of the inner electrode 4 and the inner peripheral surface of the outer electrode 5 face each other through the tubular dielectric 3.

The nozzle 1 includes a base 1b fitted in the fitting hole 2c, and a discharge tube 1c protruding from the base 1b. The base 1b and the discharge tube 1c are integrated with each other. The nozzle 1 has in its inside a second reactive gas flow path 8. The nozzle 1 has an outlet 1a at its tip end. The second reactive gas flow path 8 and the first reactive gas flow path 7 communicate with each other.

The material of the body 2b is not particularly limited, but is preferably an insulating material. Examples of the insulating material include thermoplastic resin, thermosetting resin, etc. Examples of the thermoplastic resin include polyethylene, polypropylene, polyvinyl chloride, polystyrene, acrylonitrile-butadiene-styrene resin (ABS resin), etc. Examples of the thermosetting resin include phenol resin, melamine resin, urea resin, epoxy resin, unsaturated polyester resin, silicon resin, etc.
The size of the body 2b is not particularly limited, and may be such a size that allows the body 2b to be easily grasped with fingers.

The material of the head 2a is not particularly limited, and may or may not be an insulating material. The material of the head 2a is preferably a material excellent in abrasion resistance and corrosion resistance. As an example of such a material excellent in abrasion resistance and corrosion resistance, a metal such as stainless steel can be listed. The materials of the head 2a and the body 2b may be the same or different.

The size of the head 2a can be decided in consideration of the use of the reactive gas application device 100 and the like. For example, when the reactive gas application apparatus 100 is an apparatus for an intraoral treatment, the size of the head 2a is preferably set to be such a size that allows the apparatus 100 to be inserted into an oral cavity.

As a material of the tubular dielectric 3, a dielectric material used for a known plasma generator can be employed. Examples of the material of the tubular dielectric 3 include glass, ceramics, synthetic resins, and the like. The dielectric constant of the tubular dielectric 3 is preferably as low as possible.

The inner diameter R of the tubular dielectric 3 can be appropriately decided in consideration of the outer diameter d of the inner electrode 4. The inner diameter R is set such that a distance s (described later) falls within a predetermined range.

The inner electrode 4 includes a shaft portion extending in the tube axis O1 direction and a screw thread on the outer peripheral surface of the shaft portion. The shaft portion may be solid or hollow. Of these, a solid shaft portion is more preferable. The solid shaft portion allows easy processing and improves mechanical durability. The screw thread of the inner electrode 4 is a helical screw thread that circulates in the circumferential direction of the shaft portion. The shape of the inner electrode 4 is the same as that of a screw or a bolt.

The screw thread on the outer peripheral surface of the inner electrode 4 allows the electric field at the tip of the screw thread to be locally enhanced, thereby lowering the discharge inception voltage. Therefore, plasma can be generated and maintained with less electric power.

The outer diameter d of the inner electrode 4 can be appropriately decided in consideration of the actual use of the reactive gas application apparatus 100 (that is, the size of the application instrument 10) and the like. When the reactive gas application apparatus 100 is an apparatus for an intraoral treatment, the outer diameter d is preferably 0.5 mm to 20 mm, more preferably 1 mm to 10 mm. When the outer diameter d is not less than the above lower limit value, the inner electrode 4 can be easily manufactured. Further, the outer diameter d of not less than the above lower limit value increases the surface area of the inner electrode 4, whereby plasma can be generated more efficiently, and healing and the like can be further promoted. When the outer diameter d is not more than the above upper limit value, plasma can be generated more efficiently and the healing and the like can be further promoted without excessively increasing the size of the application instrument 10.

The height h of the screw thread of the inner electrode 4 can be appropriately decided in consideration of the outer diameter d of the inner electrode 4.

The thread pitch p of the inner electrode 4 can be appropriately decided in consideration of the length and outer diameter d of the inner electrode 4, and the like.

The material of the inner electrode 4 is not particularly limited as long as the material is electrically conductive, and metals used for electrodes of known plasma generating apparatuses can be used. Examples of the material of the inner electrode 4 include metals such as stainless steel, copper and tungsten, carbon, and the like.

The inner electrode 4 preferably has the same specification as any of the metric screw threads complying with JIS B 0205: 2001 (M2, M2.2, M2.5, M3, M3.5, etc.), the metric trapezoidal screw threads complying with JIS B 2016: 1987 (Tr8 × 1.5, Tr9 × 2, Tr9 × 1.5, etc.), the unified coarse screw threads complying with JIS B 0206: 1973 (No. 1 - 64 UNC, No. 2 - 56 UNC, No. 3 - 48 UNC, etc.), and the like. The inner electrode 4 having the same specification as those standardized products is advantageous in terms of cost.

The distance s between the outer surface of the inner electrode 4 and the inner surface of the tubular dielectric 3 is preferably 0.05 mm to 5 mm, more preferably 0.1 mm to 1 mm. When the distance s is not less than the above lower limit value, a desired amount of plasma generating gas is allowed to flow easily. When the distance s is not more than the above upper limit value, plasma can be generated more efficiently and the temperature of the reactive gas can be lowered.

The material of the outer electrode 5 is not particularly limited as long as the material is electrically conductive, and metals used for electrodes of known plasma generating apparatuses can be used. Examples of the material of the outer electrode 5 include metals such as stainless steel, copper and tungsten, carbon, and the like.

The material of the nozzle 1 is not particularly limited, and may be an insulating material or a conductive material. The material of the nozzle 1 is preferably a material excellent in abrasion resistance and corrosion resistance. As an example of such a material excellent in abrasion resistance and corrosion resistance, a metal such as stainless steel can be listed.

The length (that is, the distance L2) of the flow path in the discharge tube 1c in the nozzle 1 can be appropriately decided in consideration of the use of the reactive gas application apparatus 100 or the like.

The opening diameter of the outlet 1a is preferably, for example, 0.5 mm to 5 mm. When the opening diameter is not less than the above lower limit value, the pressure loss of the reactive gas can be suppressed. When the opening diameter is not more than the above upper limit value, the flow rate of the discharged reactive gas can be increased to promote healing and the like.

The discharge tube 1c is bent with respect to the tube axis O1.

The angle θ formed between the tube axis O2 of the discharge tube 1c and the tube axis O1 can be decided in consideration of the use of the reactive gas application apparatus 100 and the like.

The sum of the distance L1 from the tip end Q1 of the inner electrode 4 to the tip end Q2 of the head 2a and the distance L2 from the tip end Q2 to the outlet 1a (that is, a distance from the inner electrode 4 to the outlet 1a) is appropriately decided in consideration of the size of the reactive gas application apparatus 100, the temperature of a surface to which the reactive gas is applied (target surface), and the like. When the sum of the distance of L1 and the distance L2 is large, the temperature of the target surface can be lowered. When the sum of the distance of L1 and the distance L2 is small, the radical concentration of the reactive gas can be further increased, and the effects of cleaning, activation, healing, etc. on the target surface can be further enhanced. The tip end Q2 is an intersection point between the tube axis O1 and the tube axis O2.

As shown in FIGs. 2, 4 and 5, the detection unit 15 is provided in the application instrument 10. As shown in FIGs. 2 and 4, the detection unit 15 detects an external force (impact force) received by the application instrument 10. The detection unit 15 is closer to the plasma generating unit 12 than the nozzle 1. When an external force is received by the application instrument 10, the tubular dielectric 3 may be damaged by collision between the tubular dielectric 3 provided in the plasma generating unit 12 and the inner electrode 4 disposed therein. Therefore, it is preferable that the detection unit 15 is provided at a position closer to the plasma generating unit 12 than the nozzle 1 to detect the external force received by the plasma generating unit 12. This makes it possible to determine whether or not the tubular dielectric material 3 is damaged.

Here, the phrase "closer to the plasma generating unit 12 than the nozzle 1" means that the distance A from the tubular dielectric 3-side end of the detector unit 15 to the tip of the tubular dielectric 3 with respect to the nozzle 1 and the plasma generating unit 12, which are separated along the tube axis O1, is shorter than the distance B from the nozzle 1-side end of the detector unit 15 to the root of the nozzle 1 (the boundary between the nozzle 1 and the cowling 2) (i.e., the ratio of distance B/distance A is less than 1). The distance A being 0 encompasses not only the case in which the position of the tubular dielectric 3-side end of the detection unit 15 and the position of the tip of the tubular dielectric 3 of the detection unit 15 coincide when viewed from the front of the detection unit 15 (i.e., viewed from the detection unit 15's surface opposite to the tubular axis O1), but also the case in which the detection unit 15 overlaps with the tubular dielectric 3.

As is evident from the above, damage to the tubular dielectric 3 is particularly likely to occur at a point where the tubular dielectric 3 and the internal electrode 4 are opposed to each other. In addition, as shown in FIG. 2, when the internal electrode 4 is shorter than the tubular dielectric 3 and the tip of the internal electrode 4 is opposed to the inner surface of the tubular dielectric 3, damage to the tubular dielectric 3 is particularly likely to occur where the tip of the internal electrode 4 is opposed to the inner surface of the tubular dielectric 3. Therefore, it is more preferable that the detection unit 15 is provided at a position where the tubular dielectric 3 is opposed to the internal electrode 4, especially where the detection unit 15 can surely detect an external force received at a position where the tip of the internal electrode 4 is opposed to the inner surface of the tubular dielectric 3. From this point of view, it is preferable for the detection unit 15 to be located at a position that overlaps the tubular dielectric 3 when the detection portion 15 is viewed from its front (i.e., the detection unit 15's surface opposite the tube axis O1), and it is more preferable for the detection unit 15 to be located at a position that overlaps the tip of the inner electrode 4.

In addition, it is necessary to place the detection unit 15 in the application instrument 10 at a position where it receives an impact equal to or greater than that received by the tubular dielectric 3. For example, it is preferable to place the detection unit 15 in a member that is continuously connected to the member that is in contact with the tubular dielectric 3 without the use of rubber such as an O-ring. Further, when the tubular dielectric 3 is disposed within the body 2b of the application instrument 10, separated from the body 2b through an O-ring or the like, it is preferable that the loss tangent of the member provided with the detection unit 15, which is positioned outside the member holding the tubular dielectric material 3, is equal to or less than the loss tangent of the material (poor shock absorption material) with which the tubular dielectric material 3 is proximate. Furthermore, it is preferable to position the detection unit 15 at a position where the impact received by the application instrument 10 can be directly detected. Specifically, a material with a velocity of elastic wave propagation inside the material of at least 3000 m/sec is placed in the outermost layer of the body 2b of the application instrument 10, and the detection unit 15 is placed in contact with such a material. As the material with a velocity of elastic wave propagation inside the material of at least 3000 m/sec, metallic materials, etc. can be used.

The detection unit 15 is disposed in the recess 16. The recess 16 is formed on the inner periphery of the body 2b. Supposing that the direction orthogonal to the tube axis O1 is in the radial direction, the detection unit 15 is located outside the tube dielectric 3 in the radial direction. The detection unit 15 is shaped in the form of a tube that extends in the direction of the tube axis O1. The tubular shape of the detection unit 15 allows the detection unit 15 to be placed in a narrow area within the application instrument 10. However, the detection unit 15 is not limited to that of a tubular shape, but can be of any shape as long as it has the function as described below.

In the context of the present specification, the term "external force" refers to the force that the application instrument 10 receives from the outside due to impact, etc. More specifically, this term refers to an impact force received by the application instrument having fallen on a floor and the like; an impact force received by the application instrument having hit a wall and the like due to pendulum motion of the application instrument dangling by wiring connected thereto; an impact force caused by a heavy object having fallen on the application instrument; and the like.

The detection unit 15 changes its color when an external force is applied to the application instrument 10. In the present embodiment, the color of the detection 15 differs between before and after the detection unit 15 receives an external force reaching or exceeding a threshold level. The color of the detection unit 15 remains the same without returning to its original color after the detection unit 15 receives an external force reaching or exceeding the threshold level.

The detection unit 15 is visible from the outside of the application instrument 10. The cowling 2 has an observation window 17. The observation window 17 is located outside of the detection unit 15 (recess 16) as viewed in its radial direction. The detection unit 15 is visible from the outside of the application instrument 10 through the observation window 17.

The supply unit 20, as shown in FIG. 1, supplies electricity and plasma generating gas to the application instrument 10. The supply unit 20 is capable of adjusting the voltage and frequency applied between the inner electrode 4 and the outer electrode 5. The supply unit 20 has a housing 21 that houses the supply source 70. The housing 21 accommodates the supply source 70 in a detachable manner. Thus, when the gas in the supply source 70 accommodated in the housing 21 runs out, the supply source 70 for plasma generating gas can be replaced.

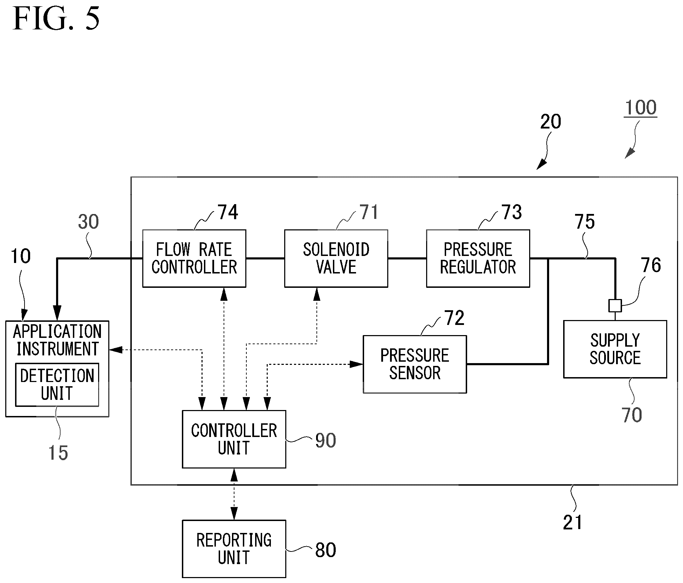

The supply source 70 supplies the plasma generating gas to the plasma generating unit 12. The supply source 70 is a pressure-resistant vessel filled with the plasma generating gas. As shown in FIG. 5, the supply source 70 is detachably attached to the pipe 75 disposed in the housing 21. The pipe 75 connects the supply source 70 with the gas conduit 30. For example, a replaceable cylinder (gas cylinder) can be used as the supply source 70.

A solenoid valve 71, a pressure regulator 73, a flow rate controller 74, and a pressure sensor 72 (residual volume sensor) are attached to the pipe 75.

When the solenoid valve 71 is opened, the plasma generating gas is supplied from the supply source 70 to the application instrument 10 through pipe 75 and gas conduit 30. In the example shown in the drawing, the solenoid valve 71 is not configured to enable adjustment of the valve opening degree, but is configured to enable only switch between opening and closing. However, the solenoid valve 71 may also be configured to enable adjustment of the valve opening degree.

The pressure regulator 73 is positioned between the solenoid valve 71 and the supply source 70. The pressure regulator 73 lowers the pressure of the plasma generating gas from the supply source 70 to the solenoid valve 71 (i.e., the pressure regulator 73 reduces the pressure of the plasma generating gas).

The flow rate controller 74 is disposed between the solenoid valve 71 and the gas conduit 30. The flow rate controller 74 adjusts the flow rate (supply rate per unit time) of the plasma generating gas having passed through the solenoid valve 71. For example, the flow rate controller 74 adjusts the flow rate of the plasma generating gas to 3 L/min.

The pressure sensor 72 measures the remaining amount of plasma generating gas V1 in the supply source 70. The pressure sensor 72 measures the remaining amount V1 in terms of the pressure (remaining pressure) in the supply source 70. The pressure sensor 72 measures the pressure (upstream pressure) of the plasma generating gas passing between the pressure regulator 73 and the supply source 70 (positioned upstream of the pressure regulator 73) as the pressure of the supply source 70. As the pressure sensor 72, for example, the AP-V80 series (e.g., AP-15S) manufactured by Keyence Corporation can be employed.

The remaining amount V1 (volume) at the supply source 70 is calculated from the remaining pressure measured by the pressure sensor 72 and the capacity (internal volume) of the supply source 70.

Assuming that supply sources 70 of various capacities are used as the supply source 70, for example, the capacity for the calculation may be set by selecting the capacity of the actual supply source 70 on the system screen of the input section not shown.

Alternatively, when supply sources 70 of the same capacity are used as the supply source 70, the capacity may be input into and stored in the controller unit 90 in advance.

A joint 76 is provided at the end of pipe 75 on the supply source 70-side. The supply source 70 is detachably attached to the joint 76. The attachment or detachment of the supply source 70 to or from the joint 76 allows for replacement of the supply source 70 for the plasma generating gas while leaving the solenoid valve 71, the pressure regulator 73, the flow rate controller 74, and the pressure sensor 72 (hereinafter, collectively referred to as "solenoid valve 71, etc.") fixed to the housing 21. In this case, a common solenoid valve 71, etc. can be used for both the old and new supply sources 70 before and after the replacement. In addition, the solenoid valve 71, etc. may be integrally fixed to the supply source 70 so as to detachable from the housing 21 together with the supply source 70.

The supply source 70 may include two or more cylinders, which respectively supply different plasma generating gases to the plasma generating unit 12. In this instance, it is possible to improve the accuracy of the remaining gas information of each cylinder by having the reporting unit 80 report the remaining gas information on the remaining number of times or retaining time for allowing each cylinder to supply the plasma generating gas to the plasma generating unit 12.

When the supply source 70 includes two or more cylinders, the reactive gas application apparatus 100 may have reporting units respectively corresponding to the cylinders. In other words, the reactive gas application apparatus 100 may be provided with the same number of reporting units 80 as the number of cylinders.

As shown in FIG. 1, the gas conduit 30 forms a path for supplying the plasma generating gas from the supply unit 20 to the application instrument 10. The gas conduit 30 is connected to the rear end of the tubular dielectric 3 of the application instrument 10. The material of the gas conduit 30 is not particularly limited, and a material used for known gas pipes can be used. Concerning a material of the gas conduit 30, a resin pipe, a rubber tube and the like can be listed as examples, and a material having flexibility is preferable.

The electrical wiring 40 is a wiring for supplying electricity from the power supply unit 20 to the application instrument 10. The electric wiring 40 is connected to the inner electrode 4, the outer electrode 5 and the operation switch 9 of the application instrument 10. The material of the electric wiring 40 is not particularly limited, and a material used for a known electric wiring can be employed. As examples of the material of the electric wiring 40, a metal lead wire covered with an insulating material and the like can be mentioned.

The controller unit 90 as shown in FIG. 5 is composed of an information processing unit. In other words, the controller unit 90 is equipped with a CPU (central processing unit), a memory and an auxiliary storage device, which are connected by buses. The controller unit 90 operates by executing a program. The controller unit 90 may, for example, be built into the supply unit 20. The controller unit 90 controls the application instrument 10, the supply unit 20, and the reporting unit 80.

An operation switch 9 for the application instrument 10 is electrically connected to the controller unit 90. When the operation switch 9 is turned on, an electrical signal is sent from the operation switch 9 to the controller unit 90. When the controller unit 90 receives the electrical signal, the controller unit 90 activates the solenoid valve 71 and the flow rate controller 74, and applies a voltage between the inner electrode 4 and the outer electrode 5.

In the present embodiment, when the operation switch 9 is a push button and the user pushes the operation switch 9 once (i.e., when the user has turned on the operation switch 9), the controller unit 90 receives the electrical signal described above. Then, the controller unit 90 opens the solenoid valve 71 for a predetermined period of time to allow the flow rate controller 74 to adjust the flow rate of the plasma generating gas having passed through the solenoid valve 71, and applies a voltage between the inner electrode 4 and the outer electrode 5 for a predetermined period of time. As a result, a predetermined amount of plasma generating gas is supplied to the plasma generating unit 12 from the supply source 70, and the reactive gas is continuously discharged from the nozzle 1 for a predetermined period of time (e.g., several seconds to several tens of seconds, or 30 seconds in the present embodiment).

That is, in the present embodiment, the amount of reactive gas discharged per one push of the operation switch 9 by the user is fixed. Such an operation for discharging a predetermined amount of reactive gas is defined as unit operation. In the present embodiment, the unit operation is a single push of the operation switch 9 by the user. The discharge amount of reactive gas per unit operation (the amount of plasma generating gas supplied from the supply source 70 to the plasma generating unit 12 per unit operation) may be a fixed value set beforehand, or may be a variable value that can be set by input through an operation panel not shown, etc.

The controller unit 90 calculates at least one of the remaining number of times N and the remaining time T for supplying the plasma generating gas to provide the remaining gas information. In the present embodiment, as the remaining gas information which may either be the remaining number of times N or the remaining time T, the controller unit 90 calculates only the remaining number of times N.

The remaining number of times N is the number of remaining unit operations allowed for the supply source 70 to supply the plasma generating gas to the plasma generating unit 12, based on the amount of the plasma generating gas remaining in the supply source 70. The remaining time T is the time allowed for the supply source 70 to supply the plasma generating gas to the plasma generating unit 12, based on the amount of the plasma generating gas remaining in the supply source 70. Further, it is necessary to stop the use of the supply source 70 while leaving some internal pressure (gas pressure) in the supply source 70 in order to avoid a decrease in workability for refilling the plasma generating gas into the supply source 70. Therefore, the remaining number of times N is set to be less than the remaining number of times supposed to be allowed for the supply source 70 to supply the plasma generating gas until the gas is completely consumed to generate plasma. Likewise, the remaining time T is set to be shorter than the remaining time supposed to be allowed for the supply source 70 to supply the plasma generating gas until the gas is completely consumed to generate plasma.

Both the remaining number of times N and the remaining time T can be calculated from the remaining amount V1 of the plasma generating gas in the supply source 70.

The remaining number of times N can be calculated, based on the remaining amount V1 and the supply amount V2 of the plasma generating gas per unit operation triggered by the operation switch 9 (that is, N = V1/V2). Specifically, the remaining number of times N is calculated by calculating the average value V2 of the amount of the plasma generating gas used (supply amount) for the latest several runs of operation, and dividing the average value V2 by the remaining amount V1 of the plasma generating gas.

The remaining time T can be calculated, based on the remaining amount V1 and the supply amount V3 of the plasma generating gas supplied from the supply source 70 to the plasma generating unit 12 per unit time (that is, T = V1/V3).

The reporting unit 80 reports at least one of the remaining number of times N and the remaining time T. In the present embodiment, the reporting unit 80 displays the remaining number of times N. The reporting unit 80 displays the remaining number of times N as a number calculated by the controller unit 90. For example, the reporting unit 80 may be a display device capable of displaying arbitrary numbers, or a mechanical counter.

In the example shown in the drawing, the reporting unit 80 is integrally provided with the housing 21 on the outer surface thereof, but may be provided independently of the supply unit 20. Further, the reporting unit 80 may display the remaining number of times N in a form other than numbers. For example, the reporting unit 80 may have a configuration that provides an analog display formed by a dial and a hand. Furthermore, for example, the reporting unit 80 may report the remaining number of times N by means of color display or lighting. In this instance, for example, it is conceivable to divide the remaining number of times N into multiple stages in advance. Specifically, for example, the display color may be changed at the respective stages (e.g., blue when the remaining number of times N is sufficiently high, yellow when the remaining number of times N is low, red when the remaining number of times N is very low, etc.). Alternatively, lighting and blinking may be switched at respective stages (e.g., constant lighting when the remaining number of times N is sufficiently high, long blinking when the remaining number of times N is low, short blinking when the remaining number of times N is very low, etc.).

Further, the reporting unit 80 may notify the remaining number N by voice. In this instance, for example, the reporting unit 80 may be a speaker. Further, in this instance, the remaining number of times N may be read out as numbers. Alternatively, the reporting unit 80 may be configured to set off an alarm sound or the like when the remaining number of times N reaches or goes below a predetermined threshold or becomes 0. It is also possible to combine the above-mentioned display of the remaining number of times N by means of numbers, etc. with the above-mentioned notification of the remaining number of times N by means of voice or alarm sounds, etc. Such a combination enables the user to recognize the remaining number of times N more quickly.

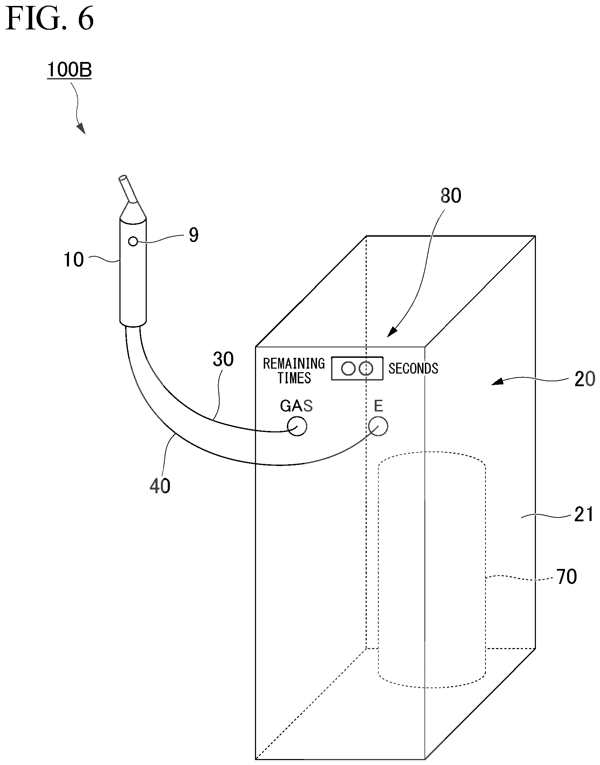

As in the present embodiment, when a predetermined amount of the plasma generating gas is supplied from the supply source 70 to the plasma generating unit 12 when the user turns on the operation switch 9, it is more convenient for the user to have the reporting unit report the remaining number of times N than the remaining time T. Unlike the present embodiment, for example, in the case of a configuration in which the plasma generating gas is continuously supplied to the plasma generating unit 12 while the operation switch 9 is being pressed down by the user, it is more convenient for the user to have the reporting unit report the remaining time T as in the case of the reactive gas application apparatus 100B of the modified example shown in FIG. 6 than the remaining number of times N. Even when the user knows the remaining gas pressure of the gas for plasma generation in the supply source 70, the remaining time T is reported as long as the user does not know the remaining number of times N.

When the controller unit 90 is connectable to a telecommunication line, the controller unit 90 may be configured to place an order for a new supply source 70 through the telecommunication line when the remaining number of times N or the remaining time T reaches or goes below a predetermined threshold.

Next, a method of using the reactive gas application apparatus 100 will be described.

A user, such as a doctor, holds and moves the application instrument 10, and points the nozzle 1 at a target object to be described later. In this state, the operation switch 9 is pushed to supply electricity and the plasma generating gas to the application instrument 10 from the supply source 70.

The plasma generating gas supplied to the application instrument 10 is allowed to flow into the hollow portion of the tubular dielectric 3 from the rear end of the tubular dielectric 3. The plasma generating gas is ionized at a position where the inner electrode 4 and the outer electrode 5 face each other, and turned into plasma.

In the present embodiment, the inner electrode 4 and the outer electrode 5 face each other in a direction orthogonal to the flowing direction of the plasma generating gas. Plasma generated at a position where the outer peripheral surface of the inner electrode 4 and the inner peripheral surface of the outer electrode 5 face each other is allowed to pass through the gas flow path 6, the first reactive gas flow path 7, and the second reactive gas flow path 8 in this order. In this process, the plasma flows while changing the gas composition, and becomes a reactive gas containing reactive species such as radicals.

The generated reactive gas is discharged from the outlet 1a. The discharged reactive gas further activates a part of the gas in the vicinity of the outlet 1a into reactive species. The reactive gas containing these reactive species is applied to a target object.

Examples of the target object include cells, living tissues, and whole bodies of organisms.

Examples of the living tissue include various organs such as internal organs, epithelial tissues covering the body surface and the inner surfaces of the body cavity, periodontal tissues such as gums, alveolar bone, periodontal ligament and cementum, teeth, bones and the like.

The whole bodies of organisms may be any of mammals such as humans, dogs, cats, pigs and the like; birds; fishes and the like.

Examples of the plasma generating gas include noble gases such as helium, neon, argon and krypton; nitrogen; and the like. With respect to these gases, a single type thereof may be used individually or two or more types thereof may be used in combination.

The plasma generating gas preferably contains nitrogen gas as a main component. Here, the nitrogen gas being contained as a main component means that the amount of the nitrogen gas contained in the plasma generating gas is more than 50 % by volume. More specifically, the amount of the nitrogen gas contained in the plasma generating gas is preferably more than 50 % by volume, more preferably 70 % by volume or more, still more preferably 90 % by volume to 100 % by volume. The gas component other than nitrogen in the plasma generating gas is not particularly limited, and examples thereof include oxygen and a noble gas.

When the reactive gas application apparatus 100 is an apparatus for an intraoral treatment, the plasma generating gas to be introduced into the tubular dielectric 3 preferably has an oxygen concentration of 1 % by volume or less. When the oxygen concentration is not more than the upper limit value, generation of ozone can be suppressed.

The flow rate of the plasma generating gas introduced into the tubular dielectric 3 is preferably 1 L/min to 10 L/min.
When the flow rate of the plasma generating gas introduced into the tubular dielectric 3 is not less than the above lower limit value, it becomes easy to suppress the temperature rise of a target surface of the target object. The flow rate is not more than the above upper limit value, the cleaning, activation or healing of the target object can be further promoted.

The alternating voltage applied between the inner electrode 4 and the outer electrode 5 is preferably 5 kVpp or more and 20 kVpp or less. Here, the unit "Vpp (peak-to-peak voltage)" representing the alternating voltage means a potential difference between the highest value and the lowest value of the alternating voltage waveform.

When the applied alternating voltage is not more than the above upper limit value, the temperature of the generated plasma can be kept low. When the applied alternating voltage is not less than the above lower limit value, plasma can be generated more efficiently.

The frequency of the alternating voltage applied between the inner electrode 4 and the outer electrode 5 is preferably 0.5 kHz or more and less than 20 kHz, more preferably 1 kHz or more and less than 15 kHz, even more preferably 2 kHz or more and less than 10 kHz, particularly preferably 3 kHz or more and less than 9 kHz, and most preferably 4 kHz or more and less than 8 kHz.

With the frequency of the alternating voltage set to less than the above upper limit value, the temperature of the generated plasma can be suppressed low. With the frequency of the alternating voltage set to equal or exceed the above lower limit value, plasma can be generated more efficiently.

The temperature of the reactive gas discharged from the outlet 1a of the nozzle 1 is preferably 50 °C or less, more preferably 45 °C or less, and even more preferably 40 °C or less.

When the temperature of the reactive gas discharged from the outlet 1a of the nozzle 1 is not more than the upper limit value, the temperature of the target surface can be easily adjusted to 40 °C or less. By keeping the temperature of the target surface at 40 °C or less, stimulus to the target surface can be reduced even when the target surface is an affected part.

The lower limit value of the temperature of the reactive gas discharged from the outlet 1a of the nozzle 1 is not particularly limited, and is, for example, 10 °C or more.

The temperature of the reactive gas is a temperature value of the reactive gas at the outlet 1a measured by a thermocouple.

The distance (application distance) from the outlet 1a to the target surface is preferably, for example, 0.01 mm to 10 mm. When the application distance is not less than the above lower limit value, the temperature of the target surface can be lowered, and the stimulus to the target surface can be further reduced. When the application distance is not more than the above upper limit value, the effect of healing and the like can be further enhanced.

The temperature of the target surface positioned at a distance of 1 mm or more and 10 mm or less from the outlet 1a is preferably 40 °C or less. By setting the temperature of the target surface to 40 °C or less, stimulus to the target surface can be reduced. The lower limit value of the temperature of the target surface is not particularly limited, and is, for example, 10 °C or more.

The temperature of the target surface is adjusted by controlling the alternating voltage applied between the inner electrode 4 and the outer electrode 5, the discharge amount of the reactive gas, the distance from the tip end Q1 of the inner electrode 4 to the outlet 1a, and the like, some or all of which are controlled in combination.

The temperature of the target surface can be measured by a thermocouple.

Examples of the reactive species (radicals etc.) contained in the reactive gas include hydroxyl radicals, singlet oxygen, ozone, hydrogen peroxide, superoxide anion radicals, nitric oxide, nitrogen dioxide, peroxynitrite, dinitrogen trioxide and the like. The type of the reactive species contained in the reactive gas can be further controlled by, for example, the type of the plasma generating gas.

The hydroxyl radical concentration of the reactive gas (radical concentration) is preferably 0.1 µmol/L to 300 µmol/L. When the radical concentration is not less than the lower limit value, the promotion of cleaning, activation or healing of a target object selected from a cell, a living tissue and a whole body of an organism is facilitated. When the radical concentration is not more than the upper limit value, stimulus to the target surface can be reduced.

The radical concentration can be measured, for example, by the following method.

A reactive gas is applied to 0.2 mL of a 0.2 mol/L solution of DMPO (5,5-dimethyl-1-pyrroline-N-oxide) for 30 seconds. Here, the distance from the outlet 1a to a liquid surface of the solution is set to 5.0 mm. With respect to the solution to which the reactive gas has been applied, a hydroxyl radical concentration is measured by electron spin resonance (ESR) method.

The singlet oxygen concentration of the reactive gas is preferably 0.1 µmol/L to 300 µmol/L. When the singlet oxygen concentration is not less than the lower limit value, the promotion of cleaning, activation or healing of a target object such as a cell, a living tissue or a whole body of an organism is facilitated. When the singlet oxygen concentration is not more than the upper limit value, stimulus to the target surface can be reduced.

The singlet oxygen concentration can be measured, for example, by the following method.

A reactive gas is applied to 0.4 mL of a 0.1 mol/L solution of TPC (2,2,5,5-tetramethyl-3-pyrroline-3-carboxamide) for 30 seconds. Here, the distance from the outlet 1a to a liquid surface of the solution is set to 5.0 mm. With respect to the solution to which the reactive gas has been applied, a singlet oxygen concentration is measured by electron spin resonance (ESR) method.

The flow rate of the reactive gas discharged from the outlet 1a is preferably 1 L/min to 10 L/min.

When the flow rate of the reactive gas discharged from the outlet 1a is not less than the above lower limit value, the effect of the reactive gas acting on the target surface can be sufficiently enhanced. When the flow rate of the reactive gas discharged from the outlet 1a is less than the above upper limit value, excessive increase in the temperature of the reactive gas at the target surface can be prevented. In addition, when the target surface is wet, rapid drying of the target surface can be prevented. Furthermore, when the target surface is an affected part of a patient, stimulus inflicted on the patient can be further suppressed.

In the reactive gas application apparatus 100, the flow rate of the reactive gas discharged from the outlet 1a can be adjusted by the supply amount of the plasma generating gas to the tubular dielectric 3.

The reactive gas generated by the reactive gas application apparatus 100 has an effect of promoting healing of trauma and other abnormalities. The application of the reactive gas to a cell, a living tissue or a whole body of an organism can promote cleaning, activation or healing of the target part to which the reactive gas is applied.

For applying a reactive gas for the purpose of promoting healing of trauma and other abnormalities, there is no particular limitation with regard to the interval, repetition number and duration of the application. For example, when a reactive gas is applied to an affected part at a dose of 1 L/min to 5.0 L/min, the application conditions preferred for promoting healing are as follows: 1 to 5 times per day, 10 seconds to 10 minutes for each repetition, and 1 to 30 days as total duration of treatment.

The reactive gas application apparatus 100 of the present embodiment is useful especially as an oral cavity treatment apparatus or a dental treatment apparatus. Further, the reactive gas application apparatus 100 of the present embodiment is also suitable as an animal treatment apparatus.

According to the reactive gas application apparatus 100 of the present embodiment as described above, the reporting unit 80 reports the remaining number of times N for supplying the plasma generating gas. Therefore, for example, the user can easily tell the timing of replacement of the supply source 70, and the usability of the plasma application therapeutic apparatus 100 can be improved. The supply source 70 is replaceable, and the plasma generating gas results in being wasted if the supply source 70 is replaced while the plasma generating gas is still remaining in the supply source 70. According to the reactive gas application apparatus 100 of the present embodiment, the user can easily tell the timing of replacing the supply source 70, so that the supply source 70 can be replaced after the plasma generating gas has been completely consumed.

The reporting unit 80 displays the remaining number of times N. Therefore, for example, the user can see the information on the remaining number of times N for supplying plasma generating gas, unlike the case in which the reporting unit 80 announces the remaining number of times N by voice.

The controller unit 90 calculates the remaining number of times N for supplying the plasma generating gas, based on the remaining amount (VI) of the plasma generating gas in the supply source 70 and the supply amount (V2) of the plasma generating gas per unit operation triggered by the operation switch 9. Therefore, the accuracy of the remaining number of times N to be reported can be increased.

In addition, the reactive gas application apparatus 100 of the present embodiment can also detect the leakage of the plasma generating gas. For example, the leakage of the plasma generating gas is detected by checking the pressure difference of the plasma generating gas at the supply source 70 from the pressure before use, the pressure after use, and the record of use on that day.

### <Other Embodiments>

The present invention is not limited to the above embodiment.

The detection unit 15 may be omitted.

The operation switch 9 may be different from the above embodiment. For example, the supply unit 20 may be provided with a foot pedal, instead of providing an operation switch 9 in the application instrument 10. In this instance, a foot pedal can be used as an operation unit and, for example, it is possible to employ a configuration in which the plasma generating gas is supplied to the plasma generating unit 12 from the supply source 70 when the user steps on the foot pedal.

The controller unit 90 may be configured to calculate the remaining number of times N without relying on the remaining amount (VI) of the plasma generating gas in the supply source 70 and the supply amount (V2) of the plasma generating gas per unit operation triggered by the operation switch 9. For example, the controller unit 90 may be configured to calculate the remaining number of times N by previously storing the input number of times N1 for the new supply source 70, and storing the input cumulative number of times N2 (cumulative discharge times) of turning on the operation switch 9 after starting to use the new supply source 70 (that is, N = N1 - N2).

The method as described above which measures the remaining amount V1 of the plasma generating gas in the supply source 70 using a pressure sensor 72 (i.e., method of calculating the remaining amount by monitoring the primary pressure with the pressure sensor 72) is preferable because it allows for more accurate determination of the remaining amount V1 in the supply source 70.

However, the method of measuring the remaining amount V1 is not limited to this method, and the remaining amount V1 may be calculated without using the pressure sensor 72. For example, the controller unit 90 may count the number of times the unit operation has been performed and calculate the remaining amount V1 by subtraction from the initial gas amount. Further, the remaining amount V1 may be calculated by calculating the amount of the already used plasma generating gas by multiplying the set value of the flow rate controller 74 by an operation time, and subtracting this amount of the used gas from the amount of the plasma generating gas in a new supply source 70. These calculations can be performed, for example, by the controller unit 90. Also, in this instance, the pipe 75 can be simplified by dispensing with the pressure sensor 72, for example by directly connecting the supply source 70 to the pressure regulator 73 (regulator). As a result, for example, the efficiency in operation for replacement of the supply source 70 can be improved. In addition, a metal pipe may be employed as the pipe 75 to improve the pressure resistance of the pipe 75.

The shape of the inner electrode 4 of the present embodiment described above is a screw shape. However, the shape of the inner electrode is not limited as long as plasma can be generated between the inner electrode and the outer electrode.

The inner electrode may or may not have concavities and convexities on its surface. However, the inner electrode preferably has concavities and convexities on the outer peripheral surface.

For example, the shape of the inner electrode may be a coil shape, or may be a rod shape or a cylindrical shape in which a plurality of protrusions, holes, and through holes are formed on the outer peripheral surface. The cross-sectional shape of the inner electrode is not particularly limited, and may be, for example, a circular shape such as a true circle or an ellipse, or a polygonal shape such as a square or a hexagon.

The features of the embodiments described above can be appropriately replaced by known equivalents as long as such replacement does not deviate from the essence of the present invention, and the modifications described above may be combined as appropriate.

### DESCRIPTION OF THE REFERENCE SIGNS

- 1: Nozzle
- 9: Operation switch
- 10: Application instrument
- 12: Plasma generating unit
- 15: Detection unit
- 70: Supply source
- 80: Detection unit
- 90: Controller unit (calculation unit)
- 100,100B: Reactive gas application apparatus

## Claims

1. A plasma application therapeutic apparatus comprising:
a plasma generating unit,
a nozzle for discharging at least one of plasma generated by the plasma generating unit and a reactive gas generated by the plasma,
a supply source for supplying a plasma generating gas to the plasma generating unit,
an operation unit which is configured to be activated by a user to allow the supply source to supply a predetermined amount of the plasma generating gas to the plasma generating unit, and
a reporting unit which is configured to report a remaining gas information in terms of remaining number of times allowed for the supply source to supply the plasma generating gas to the plasma generating unit, based on the plasma generating gas remaining in the supply source.

2. The plasma application therapeutic apparatus according to claim 1, which further comprises a calculation unit configured to calculate the remaining number of times, based on a remaining amount of the plasma generating gas in the supply source and a supply amount of the plasma generating gas per operation of the operation unit.

3. A plasma application therapeutic apparatus comprising:
a plasma generating unit,
a nozzle for discharging at least one of plasma generated by the plasma generating unit and a reactive gas generated by the plasma,
a supply source for supplying a plasma generating gas to the plasma generating unit, and
a reporting unit which is configured to report a remaining gas information in terms of remaining time allowed for the supply source to supply the plasma generating gas to the plasma generating unit, based on the plasma generating gas remaining in the supply source.

4. The plasma application therapeutic apparatus according to claim 3, which further comprises a calculation unit configured to calculate the remaining time, based on a remaining amount of the plasma generating gas in the supply source and a supply amount of the plasma generating gas per unit time.

5. The plasma treatment apparatus according to any one of claims 1 to 4, wherein the reporting unit displays the remaining gas information.

6. The plasma application therapeutic apparatus according to any one of claims 1 to 5, wherein the supply source comprises two or more cylinders which are configured to respectively supply different plasma generating gases to the plasma generating unit.
